(19) Europäisches Patentamt
European Patent Office
Office européen des brevets

(11) **EP 4 144 359 A1**

(12) **EUROPEAN PATENT APPLICATION**
published in accordance with Art. 153(4) EPC

(43) Date of publication:
**08.03.2023 Bulletin 2023/10**

(21) Application number: **21781212.2**

(22) Date of filing: **02.04.2021**

(51) International Patent Classification (IPC):
*A61K 38/16* (2006.01)  *A61P 1/04* (2006.01)
*A61P 9/10* (2006.01)  *A61P 17/00* (2006.01)
*A61P 29/00* (2006.01)  *A61P 37/02* (2006.01)
*A61P 37/08* (2006.01)  *A61P 43/00* (2006.01)
*C07K 7/08* (2006.01)  *C07K 14/00* (2006.01)

(52) Cooperative Patent Classification (CPC):
**A61K 38/16; A61P 1/04; A61P 9/10; A61P 17/00;
A61P 29/00; A61P 37/02; A61P 37/08;
A61P 43/00; C07K 7/08; C07K 14/00**

(86) International application number:
**PCT/JP2021/014253**

(87) International publication number:
**WO 2021/201260 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.04.2020 JP 2020067570**

(71) Applicants:
• **Stemrim Inc.**
**Ibaraki-shi, Osaka 567-0085 (JP)**
• **OSAKA UNIVERSITY**
**Suita-shi**
**Osaka 565-0871 (JP)**

(72) Inventors:
• **TAMAI, Katsuto**
**Suita-shi, Osaka 565-0871 (JP)**
• **SHIMBO, Takashi**
**Suita-shi, Osaka 565-0871 (JP)**
• **YAMAZAKI, Takehiko**
**Ibaraki-shi, Osaka 567-0085 (JP)**

(74) Representative: **Zacco GmbH**
**Bayerstrasse 83**
**80335 München (DE)**

Remarks:
The complete document including Reference
Table(s) and the Sequence Listing(s) can be
downloaded from the EPO website

(54) **PEPTIDE HAVING MESENCHYMAL STEM CELL MOBILIZING ACTIVITY**

(57) The present inventors synthesized a plurality of peptides each consisting of a partial sequence of the artificial sequence peptide "1r10" (1r10 fragment peptides), which was discovered from their own research conducted to date, investigated whether they have an activity of mobilizing mesenchymal stem cells into peripheral blood, and as a result, discovered that a 1r10 fragment peptide having a specific amino acid sequence shows the activity. Based on such finding, a peptide is provided which is considered to show therapeutic effects on various diseases through mobilization of mesenchymal stem cells into peripheral blood.

FIG. 1

**Description**

[Technical Field]

The present application relates to peptides having an activity of mobilizing mesenchymal stem cells, compositions for mobilizing mesenchymal stem cells, and agents for treating diseases based on the mobilization of mesenchymal stem cells.

[Background Art]

Mesenchymal stem cells contained in bone marrow fluid and the like have the ability to differentiate into various tissues (pluripotency) such as bone, cartilage, fat, muscle, nerve, and epithelium. In recent years, attempts have been widely made to perform regenerative medicine (cell transplantation therapy) using bone marrow-derived mesenchymal stem cells proliferated by culture. However, collection of bone marrow blood containing mesenchymal stem cells is done with an invasive technique which inserts a thick needle into the iliac bone repeatedly, thereby placing a large burden on the donor. In addition, mesenchymal stem cells gradually lose their proliferative ability and pluripotency when subcultured continuously in vitro. Furthermore, culturing mesenchymal stem cells based on high quality control that guarantees the safety of in vivo transplantation requires special culture equipment such as CPC (cell processing center), so the current situation is that it can be carried out only at a limited number of universities and companies.

[Citation List]

[Non-Patent Literature]

[NPL 1] PNAS 2011 Apr 19; 108 (16): 6609-14

[Patent Literature]

    [PTL 1] WO2008/053892
    [PTL 2] WO2009/133939
    [PTL 3] WO2012/147470

[Summary of Invention]

[Technical Problem]

An objective of the present application is to develop a new regenerative medicine technology that can overcome the problems of cell transplantation therapy.

[Solution to Problem]

So far, based on their own research results, the present inventors have discovered an artificial sequence peptide "1r10" that has an activity of mobilizing mesenchymal stem cells into peripheral blood, and have filed a patent application on this peptide (PCT/JP2019/039232). This time, the present inventors synthesized a plurality of peptides each consisting of a partial sequence of the artificial sequence peptide "1r10" (hereinafter also referred to as "1r10 fragment peptides"), investigated whether they have an activity of mobilizing mesenchymal stem cells into peripheral blood, and as a result, discovered that 1r10 fragment peptides having a specific sequence present the activity. Based on such findings, the present application provides peptides considered to show therapeutic effects on various diseases through mobilization of mesenchymal stem cells into peripheral blood.

Specifically, the present application provides the following:

[1] A composition for use in mobilizing mesenchymal stem cells into peripheral blood, which comprises a peptide selected from the following:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[2] A composition for use in treatment of a disease or pathological condition in a subject by mobilizing mesenchymal stem cells into peripheral blood, which comprises a peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[3] The composition of [2], wherein the treatment of a disease or pathological condition is selected from inflammation-suppressing therapy, immunomodulatory therapy, tissue regeneration-inducing therapy, and tissue fibrosis-suppressing therapy.
[4] The composition of [2], wherein the disease or pathological condition is selected from an inflammatory disease, an autoimmune disease, a disease accompanied by tissue damage, ischemia, or necrosis, and a fibrotic disease.
[5] The composition of [2], wherein the disease or pathological condition is inflammatory bowel disease.
[6] The composition of [2], wherein the disease or pathological condition is ulcerative colitis.
[7] The composition of [2], wherein the disease or pathological condition is atopic dermatitis.
[8] The composition of [2], wherein the disease or pathological condition is cerebral infarction.
[9] A composition for use in treatment of a disease selected from inflammatory bowel disease, atopic dermatitis, and cerebral infarction, which comprises a peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[10] A peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[A1] A method for mobilizing mesenchymal stem cells into peripheral blood, comprising administering to a subject an effective amount of a peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[A2] A method for treating a disease or pathological condition in a subject by mobilizing mesenchymal stem cells into peripheral blood, which comprises administering to the subject an effective amount of a peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[A3] The method of [A2], wherein the treatment of a disease or pathological condition is selected from inflammation-suppressing therapy, immunomodulatory therapy, tissue regeneration-inducing therapy, and tissue fibrosis-suppressing therapy.
[A4] The method of [A2], wherein the disease or pathological condition is selected from an inflammatory disease, an autoimmune disease, a disease accompanied by tissue damage, ischemia, or necrosis, and a fibrotic disease.
[A5] The method of [A2], wherein the disease or pathological condition is inflammatory bowel disease.
[A6] The method of [A2], wherein the disease or pathological condition is ulcerative colitis.
[A7] The method of [A2], wherein the disease or pathological condition is atopic dermatitis.
[A8] The method of [A2], wherein the disease or pathological condition is cerebral infarction.
[A9] A method for treating a disease selected from inflammatory bowel disease, atopic dermatitis, and cerebral infarction in a subject, which comprises administering to the subject an effective amount of a peptide selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[B1] A peptide for use in mobilizing mesenchymal stem cells into peripheral blood, which is selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[B2] A peptide for use in treatment of a disease or pathological condition in a subject by mobilizing mesenchymal stem cells into peripheral blood, which is selected from the following:

(a) a peptide consisting of a portion of the amino acid sequence of SEQ ID NO: 1, and comprising the amino acid sequence of SEQ ID NO: 17 or 19;
(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[B3] The peptide of [B2], wherein the treatment of a disease or pathological condition is selected from inflammation-suppressing therapy, immunomodulatory therapy, tissue regeneration-inducing therapy, and tissue fibrosis-suppressing therapy.

[B4] The peptide of [B2], wherein the disease or pathological condition is selected from an inflammatory disease, an autoimmune disease, a disease accompanied by tissue damage, ischemia, or necrosis, and a fibrotic disease.

[B5] The peptide of [B2], wherein the disease or pathological condition is inflammatory bowel disease.

[B6] The peptide of [B2], wherein the disease or pathological condition is ulcerative colitis.

[B7] The peptide of [B2], wherein the disease or pathological condition is atopic dermatitis.

[B8] The peptide of [B2], wherein the disease or pathological condition is cerebral infarction.

[B9] A peptide for use in treatment of a disease selected from inflammatory bowel disease, atopic dermatitis, and cerebral infarction, which is selected from the following:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[C1] Use of a peptide selected from the following in manufacture of a medicament or a reagent for mobilizing mesenchymal stem cells into peripheral blood:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[C2] Use of a peptide selected from the following in manufacture of a medicament for treating a disease or pathological condition in a subject by mobilizing mesenchymal stem cells into peripheral blood:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[C3] The use of [C2], wherein the treatment of a disease or pathological condition is selected from inflammation-suppressing therapy, immunomodulatory therapy, tissue regeneration-inducing therapy, and tissue fibrosis-suppressing therapy.

[C4] The use of [C2], wherein the disease or pathological condition is selected from an inflammatory disease, an autoimmune disease, a disease accompanied by tissue damage, ischemia, or necrosis, and a fibrotic disease.

[C5] The use of [C2], wherein the disease or pathological condition is inflammatory bowel disease.

[C6] The use of [C2], wherein the disease or pathological condition is ulcerative colitis.

[C7] The use of [C2], wherein the disease or pathological condition is atopic dermatitis.

[C8] The use of [C2], wherein the disease or pathological condition is cerebral infarction.

[C9] Use of a peptide selected from the following in manufacture of a medicament for treating a disease selected from inflammatory bowel disease, atopic dermatitis, and cerebral infarction:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

[Brief Description of Drawings]

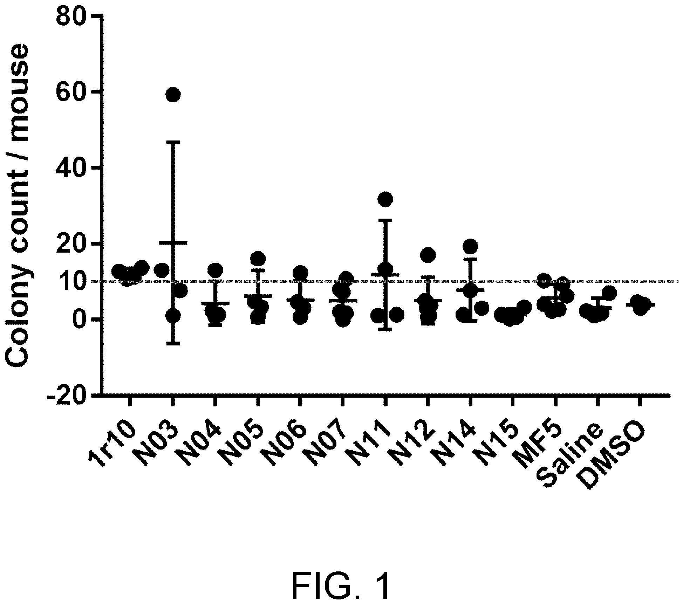

[Fig. 1] Fig. 1 is a graph produced by plotting the number of colonies obtained by culturing peripheral blood collected 16 hours after administration of physiological saline solution, 1% DMSO, or a peptide. In the graph, "Saline" and "DMSO" indicate the control groups, and the others indicate the respective peptide administration groups. The number of colonies is shown as the value per peripheral blood volume (800 $\mu$L) collected from one mouse. Regarding the error bars, the long horizontal bar represents the mean value, and the short horizontal bar represents the standard deviation. The dashed line represents the threshold value (number of colonies: 10) for determining the presence or absence of activity.

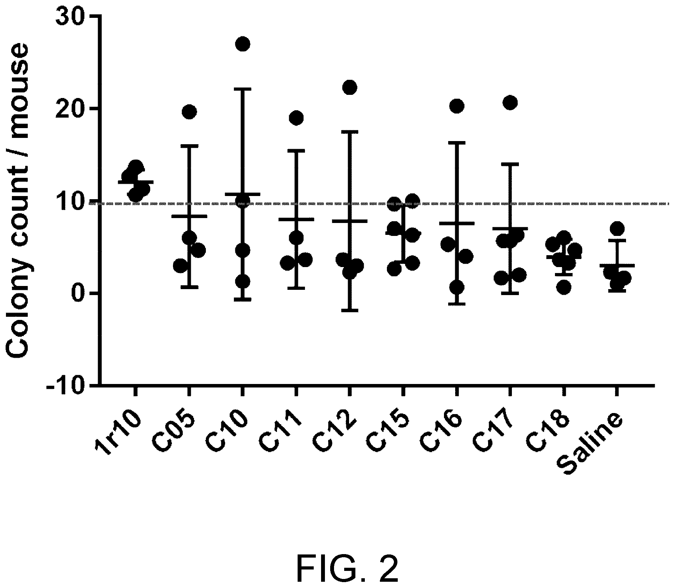

[Fig. 2] Fig. 2 is a graph produced by plotting the number of colonies obtained by culturing peripheral blood collected 16 hours after administration of physiological saline solution or a peptide. In the graph, "Saline" indicates the control group, and the others indicate the respective peptide administration groups. The number of colonies is shown as the value per peripheral blood volume (800 $\mu$L) collected from one mouse. Regarding the error bars, the long horizontal bar represents the mean value, and the short horizontal bar represents the standard deviation. The dashed line represents the threshold value (number of colonies: 10) for determining the presence or absence of activity.

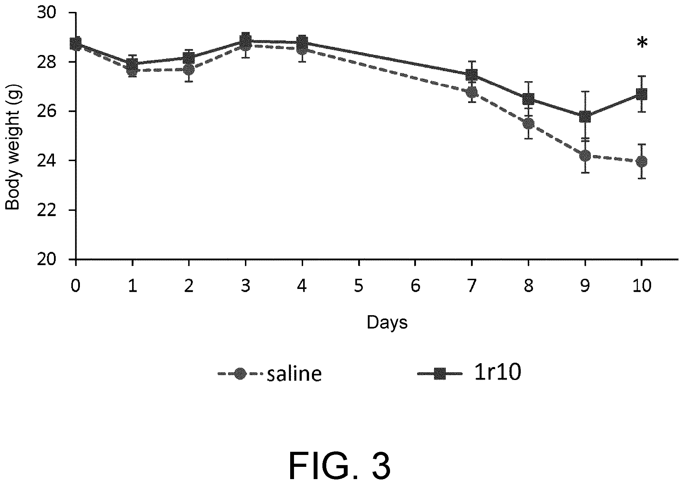

[Fig. 3] Fig. 3 is a graph showing changes in mouse body weight (mean $\pm$ standard error (SEM); * $p < 0.05$ (Student's t test)). In the graph, "saline" indicates the control group, and "1r10" indicates the peptide 1r10 administration group. The horizontal axis shows the number of days after the start of drinking an aqueous solution of dextran sulfate sodium (DSS).

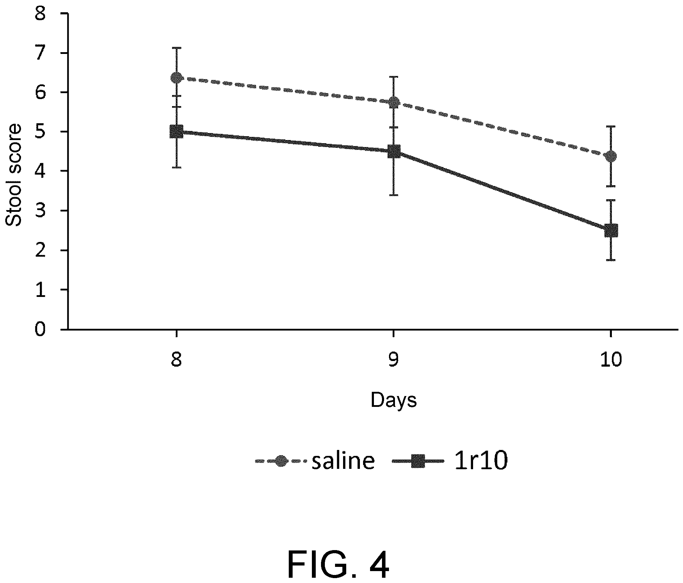

[Fig. 4] Fig. 4 is a graph showing stool scores of mice (mean $\pm$ standard error (SEM)). In the graph, "saline" indicates the control group, and " 1r10" indicates the peptide 1r10 administration group. The horizontal axis shows the number of days after the start of drinking an aqueous solution of dextran sulfate sodium (DSS).

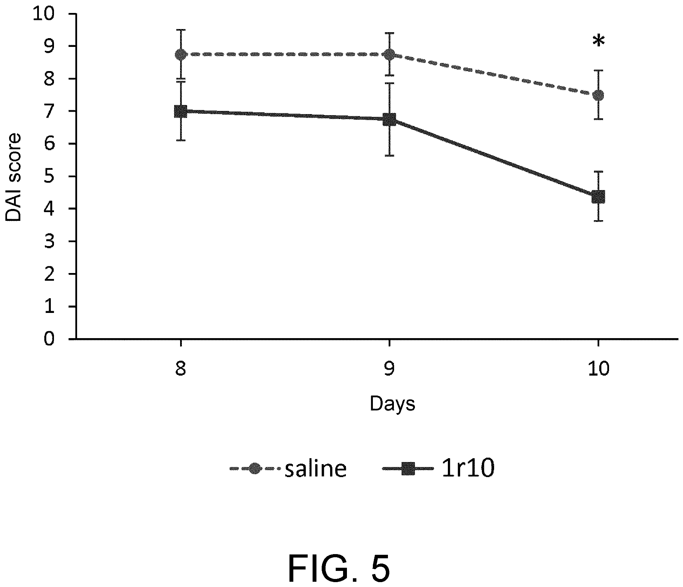

[Fig. 5] Fig. 5 is a graph showing DAI scores of mice (mean $\pm$ standard error (SEM); * $p < 0.05$ (Mann Whitney U test)). In the graph, "saline" indicates the control group, and "1r10" indicates the peptide 1r10 administration group. The horizontal axis shows the number of days after the start of drinking an aqueous solution of dextran sulfate sodium (DSS).

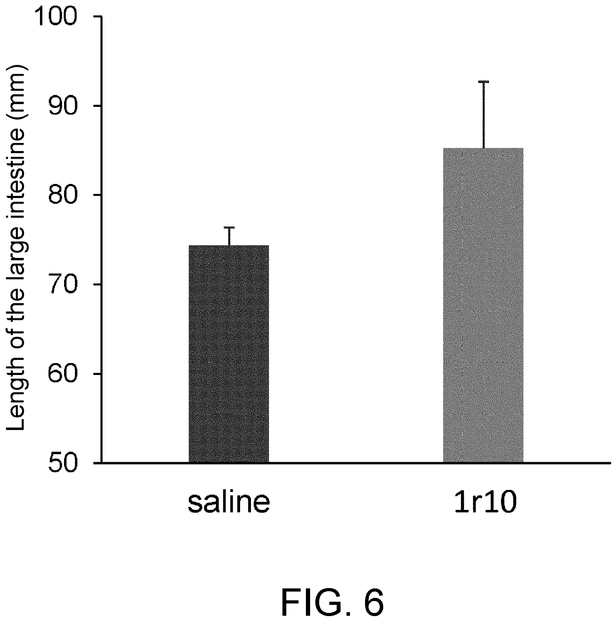

[Fig. 6] Fig. 6 is a graph showing the length of the large intestine 10 days after the start of drinking an aqueous solution of dextran sulfate sodium (DSS) (mean $\pm$ standard error (SEM)). In the graph, "saline" indicates the control group, and "1r10" indicates the peptide 1r10 administration group.

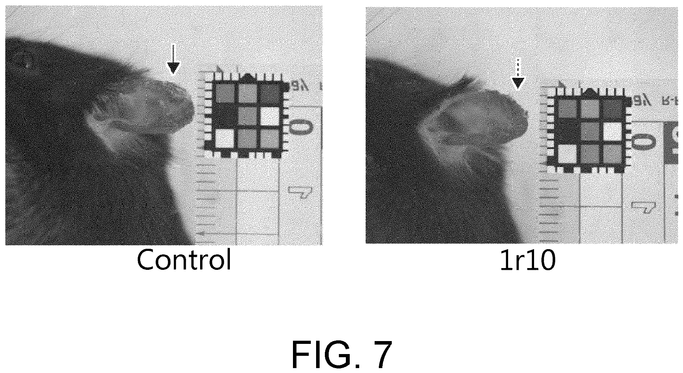

[Fig. 7] Fig. 7 presents photographs showing the auricle of mice 4 days after the start of peptide administration. Arrows indicate the rim of the auricle where scaling is likely to be seen.

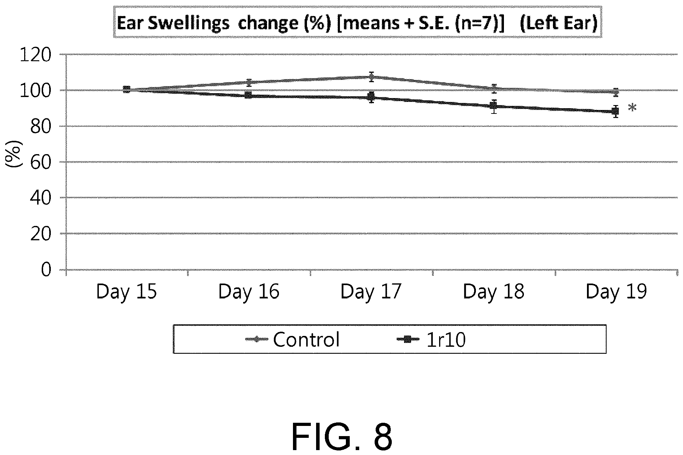

[Fig. 8] Fig. 8 is a graph showing changes in auricular thickness during the peptide administration period (the vertical axis is a relative value with the start date of peptide administration as 100%). Each point shows the mean and standard error (* $p < 0.05$; Repeated one way ANOVA test; post hoc Tukey-Kramer test).

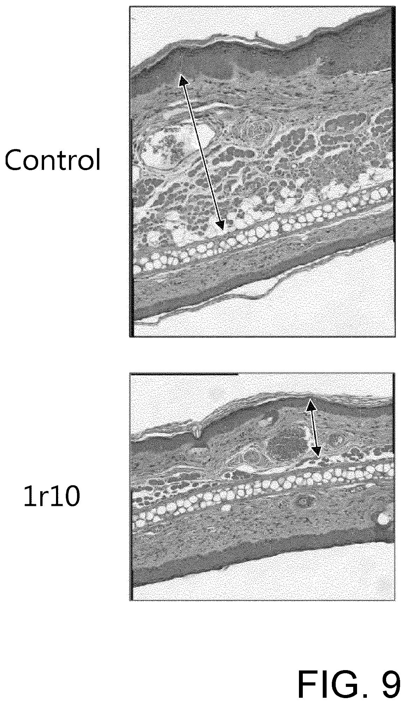

[Fig. 9] Fig. 9 presents representative photographs showing H.E. stained images of skin sections of the auricle on the 4th day after the start of peptide administration. The arrows in the photographs show examples of the measured dermis thickness.

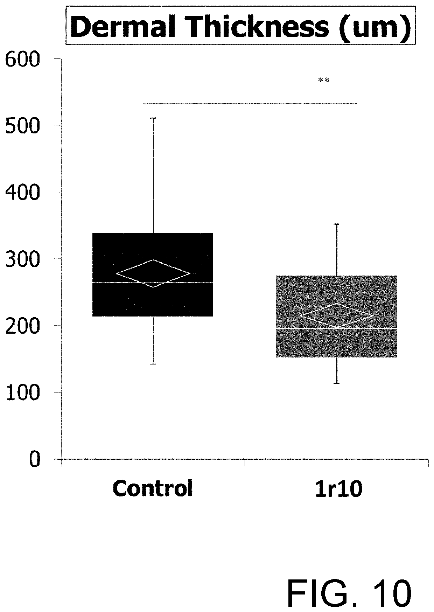

[Fig. 10] Fig. 10 is a graph (Box-Whisker plot) showing the dermis thickness of each group on the 4th day after the start of peptide administration. The central Box and Whisker indicate the median, quartile, and maximum/minimum values. Diamond in the Box shows the average value. (** $p < 0.01$ Mann Whitney U-test; 7 mice in each group x 9 measurements per mouse)

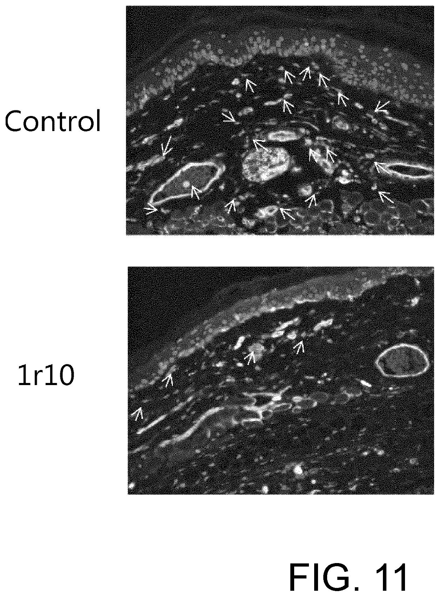

[Fig. 11] Fig. 11 presents photographs showing the multiple fluorescence immunostaining images of skin sections of the auricle on the 4th day after the start of peptide administration (Laminin (green)-CD45 (red)-DAPI (blue)).

Laminin was used to label structures in the tissue (blood vessels, basement membranes, nerves), and DAPI was used as a counterstain for the DNA of each cell.

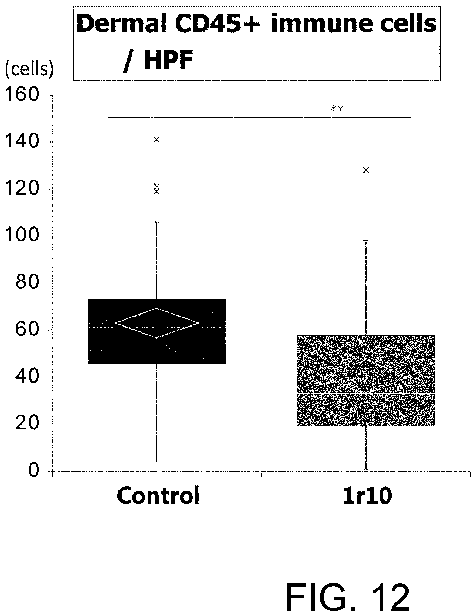

[Fig. 12] Fig. 12 is a graph (Box-Whisker plot) showing the number of CD45-positive cells (CD45+ immune cells) per unit area of high power field (400x) in each group in the auricular skin sections 4 days after the start of peptide administration. The central Box and Whisker indicate the median, quartile, and maximum/minimum values. Diamond in the Box shows the average value. "x" represents the outlier. (** p <0.01 Mann Whitney U-test; 7 mice in each group x 9 measurements per mouse. Outliers appeared in 1 out of 7 individuals (about 14%) in both the control group and the peptide-administered group. Thus, for each group, the outlier was substituted with the average value of the other 6 individuals having no outliers.)

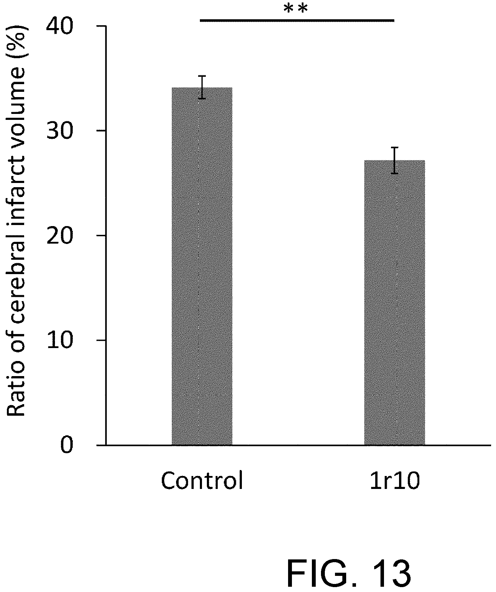

[Fig. 13] Fig. 13 is a graph showing the ratio of cerebral infarct volume 48 hours after right MCA occlusion (mean $\pm$ standard error). ** p <0.01, Mann-Whitney U test.

[Description of Embodiments]

The present inventors previously found a substance having an activity of activating stem cells in a living body or mobilizing them to an injured tissue via peripheral circulation, and believe that the substance is promising as a new type of medicine that can overcome the weaknesses of cell therapy. Specifically, the present inventors have found that High Mobility Group Box 1 (HMGB 1), which is released from necrotic tissue, mobilizes cells that are positive for platelet-derived growth factor receptor $\alpha$ (PDGFR$\alpha$), which play a role in inducing tissue regeneration in vivo (believed to be mesenchymal stem cells (MSC)), to a necrotic tissue through peripheral circulation, thereby suppresses inflammation of the necrotic tissue and promotes tissue regeneration. Further, the present inventors have also found that fragment peptides of HMGB 1 exhibit an activity of mobilizing mesenchymal stem cells into peripheral blood and a tissue regeneration-inducing activity. This time, the present inventors synthesized a plurality of peptides each consisting of a partial sequence of the previously discovered artificial sequence peptide "1r10" (hereinafter also referred to as "1r10 fragment peptides"), investigated whether they have an activity of mobilizing MSC into blood, and as a result, discovered that a 1r10 fragment peptide comprising a specific sequence presents the activity, and that the N terminal and C terminal sides of the peptide 1r10 each carry one core region that shows the activity of mobilizing MSC into blood.

It is well known to those skilled in the art that mesenchymal stem cells exert an anti-inflammatory action, immunomodulatory action, and anti-fibrotic action. In addition, since mesenchymal stem cells also have pluripotency that allows them to differentiate into various tissues, it is well known to those skilled in the art that they exert an action of promoting regeneration of injured tissues. Therefore, when a 1r10 fragment peptide having an activity of mobilizing mesenchymal stem cells into peripheral blood, or a peptide comprising the amino acid sequence of the fragment peptide and having an activity of mobilizing mesenchymal stem cells into peripheral blood is administered to a subject, the mesenchymal stem cells are recruited into the peripheral blood, and the anti-inflammatory action, immunomodulatory action, anti-fibrotic action, tissue regeneration-promoting action (due to the differentiation and/or anti-inflammatory action of mesenchymal stem cells) of the mesenchymal stem cells are considered to bring about therapeutic effects on various diseases.

The artificial sequence peptide 1r10 previously discovered by the present inventors shows therapeutic effects on inflammatory bowel disease, atopic dermatitis, and cerebral infarction, and these effects are considered to be produced through mobilization of mesenchymal stem cells into peripheral blood. Therefore, 1r10 fragment peptides having the activity of mobilizing mesenchymal stem cells into peripheral blood, or peptides comprising the amino acid sequence of the fragment peptide and having the activity of mobilizing mesenchymal stem cells into peripheral blood, are thought to show therapeutic effects on inflammatory bowel disease, atopic dermatitis, and cerebral infarction, in a similar manner to the artificial sequence peptide 1r10.

In the present application, the "artificial sequence peptide" refers to a peptide having an amino acid sequence that does not exist in nature. Further, in the present application, the "artificial sequence peptide" is also simply referred to as an "artificial peptide".

In the present application, "1r10 fragment peptide" refers to a peptide consisting of a portion of the amino acid sequence of the artificial sequence peptide 1r10 (SEQ ID NO: 1).

The present application provides compositions comprising a specific 1r10 fragment peptide or a peptide comprising the amino acid sequence of the fragment peptide, which is for use in mobilizing mesenchymal stem cells into peripheral blood.

The compositions for use in mobilizing mesenchymal stem cells into the peripheral blood of the present application can be used as a pharmaceutical composition or a reagent composition. In the present application, the term "pharmaceutical composition" is used interchangeably with "medicament", "drug" or "pharmacological composition", and the term "reagent composition" is used interchangeably with "reagent".

The compositions for use in mobilizing mesenchymal stem cells into peripheral blood of the present application can be used for treating a disease or pathological condition in a subject, for example, by mobilizing mesenchymal stem cells into peripheral blood.

Mesenchymal stem cells mobilized into peripheral blood using the composition for mobilizing mesenchymal stem cells into the peripheral blood of the present application can also be collected from the body, concentrated, and then used for treatment of a disease or pathological condition in a subject. The present application also provides use of a specific 1r10 fragment peptide or a peptide comprising the amino acid sequence of the fragment peptide in the manufacture of a medicament or a reagent for collecting mesenchymal stem cells from the body.

The compositions for use in mobilizing mesenchymal stem cells into the peripheral blood of the present application can also be used in, for example, basic research, clinical research and such. Basic research and clinical research include, but are not limited to, mesenchymal stem cell mobilization research in vitro and mesenchymal stem cell mobilization research in laboratory animals. The present application also provides use of a specific 1r10 fragment peptide or a peptide comprising the amino acid sequence of the fragment peptide (herein below these peptides may be simply referred to as "peptides") in the manufacture of a medicament or a reagent for basic or clinical research.

The compositions of the present application for use in mobilizing mesenchymal stem cells into peripheral blood can comprise one or more peptides.

In the present application, "mesenchymal stem cells" are cells having ability to differentiate into mesenchymal tissues/cells such as bone, cartilage, fat, and muscle and having self-renewal ability. In one embodiment, mesenchymal stem cells also have the ability to differentiate into epithelial tissues and nerve tissues. Mesenchymal stem cells having self-renewal ability can be discriminated, for example, by using their capability of forming colonies as an indicator. In one embodiment, mesenchymal stem cells are cells capable of forming colonies. Mesenchymal stem cells can be collected from bone marrow or other tissues (blood such as umbilical cord blood, and skin, fat, dental pulp, and such), and can be cultured and proliferated as adherent cells on solid phases such as culture dishes. In the present application, mesenchymal stem cells may exist as a heterogeneous cell population comprising not only stem cells in the narrow sense (cells having self-renewal ability and differentiation ability) but also progenitor cells. Under culture conditions, the mesenchymal stem cells may include stem cells in the narrow sense, or may even include differentiated cells in addition to stem cells in the narrow sense and progenitor cells. In one embodiment, the mesenchymal stem cells may be composed only of stem cells in the narrow sense.

In the present application, progenitor cells are defined as cells with a unidirectional ability to differentiate into cells of specific tissues other than the blood system, and include cells that have the ability to differentiate into mesenchymal tissues, epithelial tissues, nerve tissues, parenchymatous organs, vascular endothelium.

In the present application, the mesenchymal stem cells include, but are not limited to, bone marrow mesenchymal stem cells and bone marrow-derived mesenchymal stem cells. The "bone marrow mesenchymal stem cells" are mesenchymal stem cells that exist in the bone marrow. In one embodiment, bone marrow mesenchymal stem cells also have the ability to differentiate into epithelial tissues and nerve tissues. In one embodiment, bone marrow mesenchymal stem cells are cells capable of forming colonies. In the present application, the term "bone marrow mesenchymal stem cell" is used interchangeably with "bone marrow mesenchymal stromal cell", "bone marrow pluripotent stem cell" or "bone marrow pluripotent stromal cell".

"Bone marrow-derived mesenchymal stem cells" refers to mesenchymal stem cells that have been mobilized from bone marrow to the outside of the bone marrow. Bone marrow-derived mesenchymal stem cells can be collected by peripheral blood collection, and further from mesenchymal tissues such as fat, epithelial tissues such as skin, or nerve tissues such as the brain. In the present application, the term "bone marrow-derived mesenchymal stem cell" can be used interchangeably with "bone marrow-derived mesenchymal stromal cell", "bone marrow-derived pluripotent stem cell" or "bone marrow-derived pluripotent stromal cell".

In one embodiment, bone marrow mesenchymal stem cells and bone marrow-derived mesenchymal stem cells may also have the ability of, by being administered to an injured part of a living body directly after collection or after once attached to a culture dish, differentiating into, for example, epithelial tissues such as skin-constituting keratinocytes or tissues of the nerve system which constitutes the brain.

Bone marrow mesenchymal stem cells and bone marrow-derived mesenchymal stem cells may have the ability to differentiate into osteoblast cells (identifiable by calcium deposition observed when differentiation is induced), cartilage cells (identifiable by being Alcian blue staining-positive, safranin-O staining-positive, or such), and fat cells (identifiable by being Sudan III staining-positive or such), and also differentiate into, for example, mesenchymal cells such as fibroblasts, smooth muscle cells, skeletal muscle cells, stromal cells, and tendon cells, nerve cells, pigment cells, epidermal cells, hair follicle cells (expressing cytokeratin family, hair keratin family or such), epithelial cells (for example, epithelial keratinized cells and intestinal epithelial cells express cytokeratin family or such), endothelial cells, and further differentiate into cells of parenchymal organs such as liver, kidney and pancreas, but the differentiated cells are not limited to the above cells.

Human mesenchymal stem cell markers can be exemplified by some or all of PDGFRα positive, PDGFRβ positive, Lin negative, CD45 negative, CD44 positive, CD90 positive, CD29 positive, Flk-1 negative, CD105 positive, CD73 positive, CD90 positive, CD71 positive, Stro-1 positive, CD106 positive, CD166 positive, CD31 negative, CD271 positive, and CD11b negative, but are not limited thereto.

Murine mesenchymal stem cell markers can be exemplified by some or all of CD44 positive, PDGFRα positive, PDGFRβ positive, CD45 negative, Lin negative, Sca-1 positive, c-kit negative, CD90 positive, CD105 positive, CD29 positive, Flk-1 negative, CD271 positive, and CD11b negative, but are not limited thereto.

Rat mesenchymal stem cell markers can be exemplified by some or all of PDGFRα positive, CD44 positive, CD54 positive, CD73 positive, CD90 positive, CD105 positive, CD29 positive, CD271 positive, CD31 negative, and CD45 negative, but are not limited thereto.

In the present application, examples of mesenchymal stem cells include PDGFRα-positive mesenchymal stem cells, PDGFRα-positive bone marrow-derived mesenchymal stem cells, and PDGFRα-positive bone marrow-derived cells obtained as adherent cells by cell culture of a mononuclear cell fraction in blood obtained by bone marrow harvest (bone marrow cell collection) or peripheral blood collection, but they are not limited thereto. Examples of PDGFRα-positive mesenchymal stem cells include PDGFRα- and CD44-positive cells, PDGFRα- and CD90-positive cells, PDGFRα- and CD105-positive cells, PDGFRα- and CD29-positive cells, and such. In one embodiment, PDGFRα-positive mesenchymal stem cells may be CD44-negative cells.

The present application provides compositions for use in the treatment of a disease or a pathological condition in a subject by mobilizing mesenchymal stem cells into the peripheral blood, comprising a specific 1r10 fragment peptide or a peptide comprising the amino acid sequence of the fragment peptide.

The compositions for use in the treatment of a disease or a pathological condition in a subject by mobilizing mesenchymal stem cells into the peripheral blood in the present application can be used as pharmaceutical compositions.

The subject in the present application is not particularly limited, and examples thereof include mammals, birds, and fish. Mammals include human and non-human animals, for example, human, mouse, rat, monkey, pig, dog, rabbit, hamster, guinea pig, horse, sheep, and whale, but are not limited thereto. In the present application, the term "subject" is used interchangeably with "patient", "individual", or "animal".

The composition for use in the treatment of a disease or pathological condition in a subject by mobilization of mesenchymal stem cells into the peripheral blood in the present application can comprise one or more peptides.

In the present application, the treatment of a disease or pathological condition is selected from, for example, inflammation-suppressing therapy, immunomodulatory therapy, tissue regeneration-inducing therapy, and tissue fibrosis-suppressing therapy, but is not limited thereto.

In the present application, the disease or pathological condition is selected from inflammatory diseases, autoimmune diseases, diseases accompanied by tissue damage, ischemia, or necrosis, and fibrotic diseases, but is not limited thereto.

In the present application, the inflammatory disease includes, for example, inflammatory bowel disease and atopic dermatitis, but is not limited thereto. The autoimmune disease includes, for example, inflammatory bowel disease, but is not limited thereto. The fibrotic disease includes, for example, lung fibrosis, but is not limited thereto. The disease accompanied by tissue damage, ischemia, or necrosis includes, for example, inflammatory bowel disease and cerebral infarction, but is not limited thereto. The inflammatory bowel disease includes, but is not limited to, ulcerative colitis and Crohn's disease.

In the present application, the term "activity of mobilizing mesenchymal stem cells into peripheral blood" is used interchangeably with "activity to increase the abundance of mesenchymal stem cells in peripheral blood" and "activity to mobilize MSC into blood".

The activity of a peptide of the present application to mobilize MSC into blood can be assessed, for example by i) collecting peripheral blood from an individual administered with a peptide and an individual not administered with the peptide, seeding and culturing in a culture dish (for several days to 10 days), and counting the number of colonies formed; and ii) confirming that the cells which formed colonies have the ability to adhere to the solid phase and the ability to differentiate into osteoblasts, chondrocytes, and adipocytes. In step i) mentioned above, before seeding the collected peripheral blood on a culture dish, red blood cells may be removed from the peripheral blood in a desired manner. In step ii) mentioned above, instead of confirming the ability to differentiate, surface marker analysis and transcriptome analysis may be performed on cells that formed colonies to confirm that the cells have properties characteristic of mesenchymal stem cells.

The peptides of the present application can be obtained as genetic recombinants (recombinants) by incorporating DNAs encoding the peptides into an appropriate expression system, or they can be synthesized artificially. Therefore, peptides in this application include peptides prepared using cells, and artificially synthesized peptides (the so-called synthetic peptides).

In order to obtain the peptides of the present application by genetic engineering techniques, DNAs encoding the peptides may be incorporated into an appropriate expression system and expressed.

Hosts applicable to the present application include, but are not limited to, prokaryotic cells and eukaryotic cells. In addition, hosts applicable to the present application also include bacteria (e.g., *Escherichia coli*), yeast, animal cells (e.g., mammalian cells such as HEK293 cells and CHO cells, insect cells such as silkworm cells), plant cells and such, but are not limited thereto.

As the host/vector system applicable to the present application, for example, the expression vector pGEX and *Escherichia coli* can be shown. pGEX can express a foreign gene as a fusion protein with glutathione S-transferase (GST) (Gene, 67: 31-40, 1988). As such, pGEX into which DNA encoding the peptide of the present application has been incorporated is introduced into an *E. coli* strain such as BL21 by heat shock, and after culturing for an appropriate period of time, isopropylthio-β-D-galactoside (IPTG) is added to induce the expression of the GST fusion peptide. GST in the present application adsorbs to glutathione Sepharose 4B, and thus the expression product can be easily separated and purified by affinity chromatography.

In addition to this, the following can be applied as a host/vector system for obtaining a genetic recombinant of the peptide of the present application. First, when a bacterium is used as a host, a vector for expressing a fusion protein using a tag or such is commercially available. In addition, the genetic recombinant of the present application also includes those in which a tag or a partial peptide thereof is added.

The tag added to the peptide of the present application is not particularly limited as long as it does not affect the activity of the peptide of the present application, and includes, for example, a histidine tag (for example, 6x His, 10x His), HA tag, FLAG tag, GST tag, T7-tag, HSV-tag, E-tag, lck tag, and B-tag.

Among yeasts, it is known that Pichia yeast is effective for the expression of proteins having sugar chains. In terms of the addition of sugar chains, the expression system that uses a Baculovirus vector, which uses an insect cell as a host, is also useful (Bio/Technology, 6:47-55, 1988). Furthermore, mammalian cells are used for transfection with a vector that uses the promoter of CMV, RSV, SV40, or such. These host/vector systems can be used as an expression system for the peptide of the present application. In addition, plasmid vectors, retrovirus vectors, lentivirus vectors, adenovirus vectors, adeno-associated virus vectors, Sendai virus vectors, Sendai virus envelope vectors, papilloma virus vectors, and such virus vectors may also be used to introduce the gene, without limitation thereto. The vector may also contain a promoter DNA sequence that effectively induces gene expression, factors that control gene expression, and molecules necessary to maintain the stability of the DNA.

The resulting peptide in the present application can be isolated from the host cell or outside of the cell (such as medium), and purified as a substantially pure homogeneous peptide. For separation and purification of peptides, any separation and purification methods used in standard peptide purification may be utilized, without limitation. For example, chromatography columns, filters, ultrafiltration, salting out, solvent precipitation, solvent extraction, distillation, immunoprecipitation, SDS-polyacrylamide gel electrophoresis, isoelectric focusing, dialysis, recrystallization, and such are appropriately selected and combined for peptide separation and purification.

Chromatography includes, for example, affinity chromatography, ion exchange chromatography, hydrophobic chromatography, gel filtration, reverse phase chromatography, adsorption chromatography and such (Marshak et al, Strategies for Protein Purification and Characterization: A Laboratory Course Manual. Ed Daniel R. Cold Spring Harbor Laboratory Press, 1996). These chromatographies can be performed using liquid chromatography such as HPLC and FPLC.

Further, the peptide in the present application is preferably a substantially purified peptide. Here, "substantially purified" means that the degree of purification of the peptide of the present application (the ratio of the peptide of the present application in the entire protein component) is 50% or more, 60% or more, 70% or more, 80% or more, 90% or more, 95% or more, 100% or nearly 100%. The nearly 100% upper limit depends on the purification techniques and analysis techniques of those skilled in the art and may be, for example, 99.999 %, 99.99 %, 99.9 %, or 99%.

Further, the substantially purified peptide includes those purified by whatever purification method as long as they have the above purity. Examples include, but are not limited to, peptides substantially purified by appropriately selecting or combining the above-mentioned chromatography columns, filters, ultrafiltration, salting out, solvent precipitation, solvent extraction, distillation, immunoprecipitation, SDS-polyacrylamide gel electrophoresis, isoelectric focusing, dialysis, recrystallization and such.

On the other hand, the peptides in the present application can also be artificially synthesized. In the peptide synthesis method of the present application, peptides can be chemically synthesized by methods such as a peptide liquid-phase synthesis method and a peptide solid-phase synthesis method. The peptide solid-phase synthesis method is one of the methods generally used when chemically synthesizing a peptide. Polystyrene polymer gel beads having a diameter of about 0.1 mm modified with amino groups on the surface are used as a solid phase, from which the amino acid chain is extended one by one via dehydration reaction. When the sequence of the target peptide is completed, it is excised from the solid phase surface to obtain the target substance. Solid phase synthesis enables synthesis of ribosome peptides, which are difficult to synthesize in bacteria, introduction of unnatural amino acids such as D-form and stable isotope ($^2$H, $^{13}$C, $^{15}$N, etc.)-substituted amino acids, introduction of heavy atom-substituted amino acids (e.g., selenoamino acids such as selenomethionine), modification of peptide and protein main chains, and such. When synthesizing a long peptide chain of 70 to more than 100 amino acids in the solid phase method, it can be synthesized by ligating two peptide chains using the native chemical ligation method. The peptide in the present application may be in the form of a pharmaceutically acceptable salt of the peptide. Examples of pharmaceutically acceptable salts include, but are not limited to, hydrochlorides, acetates, and trifluoroacetates. The peptide in the present application may be in the form of a solvate of the peptide, or a solvate of a pharmaceutically acceptable salt of the peptide. A solvate refers to a solute molecule to which an arbitrary number of solvent molecules are coordinated, and examples thereof include hydrates, but are not limited thereto.

The amino acid length of the 1r10 fragment peptides in the present application can be, for example, in the range of 11 to 29 amino acids, such as 11, 12, 13, 14, 15, 16, 17, 18, 19, 20, 21, 22, 23, 24, 25, 26, 27, 28, or 29 amino acids.

Examples of the 1r10 fragment peptides in the present application include peptides selected from below:

(1) a peptide consisting of a portion of the amino acid sequence of SEQ ID NO: 1, and comprising the amino acid sequence of SEQ ID NO: 17 or 19;

(2) a peptide consisting of the amino acid sequence from position 1 to position 27 of SEQ ID NO: 1 (1r10_N03; SEQ ID NO: 2);

(3) a peptide consisting of the amino acid sequence from position 1 to position 26 of SEQ ID NO: 1 (1r10_N04; SEQ ID NO: 3);

(4) a peptide consisting of the amino acid sequence from position 1 to position 25 of SEQ ID NO: 1 (1r10_N05; SEQ ID NO: 4);

(5) a peptide consisting of the amino acid sequence from position 1 to position 24 of SEQ ID NO: 1 (1r10_N06; SEQ ID NO: 5);

(6) a peptide consisting of the amino acid sequence from position 1 to position 23 of SEQ ID NO: 1 (1r10_N07; SEQ ID NO: 6);

(7) a peptide consisting of the amino acid sequence from position 1 to position 19 of SEQ ID NO: 1 (1r10 N11; SEQ ID NO: 7);

(8) a peptide consisting of the amino acid sequence from position 1 to position 18 of SEQ ID NO: 1 (1r10_N12; SEQ ID NO: 8);

(9) a peptide consisting of the amino acid sequence from position 1 to position 16 of SEQ ID NO: 1 (1r10_N14; SEQ ID NO: 9);

(10) a peptide consisting of the amino acid sequence from position 6 to position 30 of SEQ ID NO: 1 (1r10_C05; SEQ ID NO: 11);

(11) a peptide consisting of the amino acid sequence from position 11 to position 30 of SEQ ID NO: 1 (1r10_C10; SEQ ID NO: 12);

(12) a peptide consisting of the amino acid sequence from position 12 to position 30 of SEQ ID NO: 1 (1r10_C11; SEQ ID NO: 13);

(13) a peptide consisting of the amino acid sequence from position 13 to position 30 of SEQ ID NO: 1 (1r10_C12; SEQ ID NO: 14);

(14) a peptide consisting of the amino acid sequence from position 16 to position 30 of SEQ ID NO: 1 (1r10_C15; SEQ ID NO: 15);

(15) a peptide consisting of the amino acid sequence from position 17 to position 30 of SEQ ID NO: 1 1r10_C16; SEQ ID NO: 16);

(16) a peptide consisting of the amino acid sequence from position 18 to position 30 of SEQ ID NO: 1 (1r10_C17; SEQ ID NO: 17); and

(17) a peptide consisting of the amino acid sequence from position 6 to position 16 of SEQ ID NO: 1 (1r10_MF5; SEQ ID NO: 19).

Among the amino acid sequences of (1) to (17) mentioned above, "the amino acid sequence from position 18 to position 30 of SEQ ID NO: 1" (the amino acid sequence of SEQ ID NO: 17) described in (16) and "the amino acid sequence from position 6 to position 16 of SEQ ID NO: 1" (the amino acid sequence of SEQ ID NO: 19) described in (17) are core regions important for 1r10 fragment peptides to retain their activity of mobilizing MSC into blood. If a 1r10 fragment peptide comprises at least one of the two core regions, its activity of mobilizing MSC into blood is retained.

"A portion of the amino acid sequence of SEQ ID NO: 1" in "a peptide consisting of a portion of the amino acid sequence of SEQ ID NO: 1, and comprising the amino acid sequence of SEQ ID NO: 17 or 19" described in (1) mentioned above includes the amino acid sequence of any one of (2) to (15) mentioned above, but is not limited thereto.

Peptides in the present application also include peptides selected from below:

(i) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(ii) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

Such peptides can be produced by methods well known to those skilled in the art.

The peptides selected from (i) and (ii) mentioned above may be, for example, a peptide consisting of not more than 30, not more than 29, not more than 28, not more than 27, not more than 26, not more than 25, not more than 24, not more than 23, not more than 22, not more than 21, not more than 20, not more than 19, not more than 18, not more than 17, not more than 16, not more than 15, not more than 14, not more than 13, not more than 12, or not more than 11 amino acids. The peptides selected from the above-mentioned (i) and (ii) may be peptides excluding the peptide consisting of the amino acid sequence of SEQ ID NO: 1.

Regarding the above-mentioned peptide of (i), examples of "one to five" include, five, four, three, two, one, one to five, one to four, one to three, or one or two.

Regarding the above-mentioned peptide of (ii), "90% or more" can also be expressed as "90% or more and less than 100%", and examples of "90% or more" include 90% or more, 91% or more, 92% or more, 93% or more, 94% or more, 95% or more, 96% or more, 97% or more, 98% or more, or 99% or more.

The 1r10 fragment peptides selected from (1) to (17) mentioned above are 1r10 fragment peptides having an activity of mobilizing mesenchymal stem cells into peripheral blood. The peptides selected from (i) and (ii) mentioned above are also peptides having an activity of mobilizing mesenchymal stem cells into peripheral blood. Therefore, these peptides are thought to have the effect of mobilizing mesenchymal stem cells into peripheral blood, as well as therapeutic effects on inflammatory diseases, autoimmune diseases, diseases accompanied by tissue damage, ischemia, or necrosis, and fibrotic diseases.

The present application also provides the peptides disclosed herein, for example, 1r10 fragment peptides selected from (1) to (17) mentioned above, and peptides selected from (i) and (ii) mentioned above.

The amino acid sequence described in SEQ ID NO: 1 is the amino acid sequence of the artificial sequence peptide 1r10 of the present application. The amino acid sequences described in SEQ ID NOs: 2 to 19 are the amino acid sequences of the 1r10 fragment peptides of the present application. The regions in the amino acid sequence described in SEQ ID NO: 1 (the amino acid sequence of 1r10) corresponding to the amino acid sequences of the 1r10 fragment peptides are shown in Table 1 below.

The nucleotide sequence described in SEQ ID NO: 20 is an example of the nucleotide sequence of DNA encoding the artificial sequence peptide 1r10 of the present application. The nucleotide sequences described in SEQ ID NOs: 21 and 22 are examples of the nucleotide sequences of DNAs encoding 1r10_C17 and 1r10_MF5, respectively, which are 1r10 fragment peptides of the present application. The regions in the nucleotide sequence of SEQ ID NO: 20 (the exemplified nucleotide sequence of 1r10) that correspond to the nucleotide sequences of DNAs encoding the 1r10 fragment peptides are shown in Table 1 below.

[Table 1]

| Peptide name | Corresponding region in the amino acid sequence of 1 r10 | Corresponding region in the exemplified nucleotide sequence of 1 r10 |
|---|---|---|
| 1r10 | 1-30 | 1-90 |
| 1r10_N03 | 1-27 | 1-81 |
| 1r10_N04 | 1-26 | 1-78 |
| 1r10_N05 | 1-25 | 1-75 |
| 1r10_N06 | 1-24 | 1-72 |
| 1r10_N07 | 1-23 | 1-69 |
| 1r10_N11 | 1-19 | 1-57 |
| 1r10_N12 | 1-18 | 1-54 |
| 1r10_N14 | 1-16 | 1-48 |
| 1r10_N15 | 1-15 | 1-45 |
| 1r10_C05 | 6-30 | 16-90 |
| 1r10_C10 | 11-30 | 31-90 |
| 1r10_C11 | 12-30 | 34-90 |
| 1r10_C12 | 13-30 | 37-90 |

(continued)

| Peptide name | Corresponding region in the amino acid sequence of 1 r10 | Corresponding region in the exemplified nucleotide sequence of 1 r10 |
|---|---|---|
| 1r10_C15 | 16-30 | 46-90 |
| 1r10_C16 | 17-30 | 49-90 |
| 1r10_C17 | 18-30 | 52-90 |
| 1r10_C18 | 19-30 | 55-90 |
| 1r10_MF5 | 6-16 | 16-48 |

Other examples of the nucleotide sequences of the DNAs encoding the artificial sequence peptide 1r10 or the 1r10 fragment peptides of the present application are apparent to those skilled in the art. The DNAs can be produced by a method of converting the amino acid residues of the artificial sequence peptide 1r10 or the 1r10 fragment peptides to corresponding codons using codon tables known to those skilled in the art (reverse translation). Reverse translation can be performed by using a variety of software (including programs, algorithms, etc.) developed for the analysis of amino acid and nucleic acid sequences as desired.

An effective amount of a peptide of the present application, or a pharmaceutical composition comprising the same (hereinafter referred to as "a pharmaceutical composition and such") is administered to a subject for the treatment of diseases and conditions described herein.

The effective amount in the present application means an amount sufficient for the treatment of the diseases or pathological conditions described herein. The treatment in the present application includes alleviation, delay, inhibition, amelioration, remission, cure, and full recovery, but are not limited thereto.

There is no limitation on the site of administration of the pharmaceutical composition and such of the present application, and the pharmaceutical composition and such of the present application can exert its effect when administered to any site, such as a site where the symptoms of the disease or pathological condition appear or a site nearby, a site different from these sites (sites other than these sites), a site separated from a site where the symptoms of the disease or pathological condition appear, a site distal to a site where the symptoms of the disease or pathological condition appear, or a site distal and ectopic to a site where the symptoms of the disease or pathological condition appear.

Further, the pharmaceutical composition and such of the present application can exert its effect when administered to any tissue, such as a tissue different from a tissue in which the symptoms of the disease or the pathological condition appear, a tissue separated from a tissue in which the symptoms of the disease or the pathological condition appear, a tissue distal to a tissue in which the symptoms of the disease or the pathological condition appear, or a tissue distal and ectopic to a tissue in which the symptoms of the disease or pathological condition appear.

Methods of administering the pharmaceutical composition and such of the present application include, but are not limited to, oral administration and parenteral administration, and methods of parenteral administration include intravascular administration (intra-arterial administration, intravenous administration, etc.), intramuscular administration, subcutaneous administration, intradermal administration, intraperitoneal administration, nasal administration, pulmonary administration, transdermal administration, and such. In addition, the pharmaceutical composition and such of the present application can be administered systemically or locally (for example, subcutaneously, intradermally, or to the skin surface, eyeball, or palpebral conjunctiva, nasal mucosa, oral and gastrointestinal mucosa, vaginal and endometrial mucosa, or injured site) by injection administration, for example, intravenous injection, intramuscular injection, intraperitoneal injection, and subcutaneous injection.

In place of the peptide of the present application, a cell secreting the peptide, a gene therapy vector into which a DNA encoding the peptide is inserted, and a pharmaceutical composition comprising them can be used.

In addition, the administration method can be appropriately selected depending on the age and symptoms of a patient. When the pharmaceutical composition and such of the present application is administered, for example, the dose can be selected from the range of 0.0000001 mg to 1000 mg per kilogram of body weight per administration. Alternatively, for example, the dose can be selected from the range of 0.00001 to 100000 mg/body per patient. When administering cells secreting the peptide of the present application or gene therapy vectors into which DNA encoding the peptide is inserted, they can be administered so that the amount of the peptide is within the above range. However, the pharmaceutical compositions in the present application are not limited to these dosages.

The pharmaceutical compositions of the present application can be formulated according to conventional methods (e.g., Remington's Pharmaceutical Science, latest edition, Mark Publishing Company, Easton, U.S.A.), and may contain pharmaceutically acceptable carriers or additives together. Examples include, but are not limited to, surfactants, excipients, coloring agents, perfumes, preservatives, stabilizers, buffers, suspending agents, isotonizing agents, binding agents, disintegrants, lubricants, fluidity-promoting agents, and flavoring agents. Other commonly used carriers can also be used as appropriate. Specific examples include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium-chain fatty acid triglyceride, polyoxyethylene hydrogenated castor oil 60, white sugar, carboxymethyl cellulose, cornstarch, and inorganic salts.

All prior art documents cited herein are incorporated herein as references.

Herein below, the present invention will be further illustrated with reference to Examples, but it is not to be construed as being limited thereto.

[Examples]

Example 1

Mobilization of mesenchymal stem cells by 1r10 fragment peptide

(1) Materials and methods

i) Peptide production

The peptides (1r10 and 1r10 fragment peptides) shown in the following Table were chemically synthesized by the solid phase method (the obtained peptides were all in the form of a trifluoroacetic acid (TFA) salt).

[Table 2]

| Peptide name | SEQ ID NO |
| --- | --- |
| 1r10 | 1 |
| 1r10_N03 | 2 |
| 1r10_N04 | 3 |
| 1r10_N05 | 4 |
| 1r10_N06 | 5 |
| 1r10_N07 | 6 |
| 1r10_N11 | 7 |
| 1r10_N12 | 8 |
| 1r10_N14 | 9 |
| 1r10_N15 | 10 |
| 1r10_C05 | 11 |
| 1r10_C10 | 12 |
| 1r10_C11 | 13 |
| 1r10_C12 | 14 |
| 1r10_C15 | 15 |
| 1r10_C16 | 16 |
| 1r10_C17 | 17 |
| 1r10_C18 | 18 |
| 1r10_MF5 | 19 |

ii) Peptide administration

C57BL/6J mice (8 weeks old, male, body weight 25 g) were prepared and divided into control groups and groups to which each of the peptides in the Table above were administered. The peptide was administered by injecting into the tail vein, a solution of each peptide adjusted to a concentration of 1 $\mu$g/$\mu$L using physiological saline or 1% DMSO as a solvent in an amount of 100 $\mu$L/animal (4 mg/kg dose of peptide) (1% DMSO was used as the solvent for 1r10_N14 and 1r10_N15, and physiological saline was used as the solvent for the other peptides). For the control groups, physiological saline or 1% DMSO was injected into the tail vein in an amount of 100 $\mu$L/animal.

iii) Cell collection from peripheral blood

Sixteen hours after the administration of physiological saline or 1% DMSO, or peptide, 800 $\mu$L per mice of peripheral blood was collected from the heart under general anesthesia (1-mL syringe containing heparin was used). For each of the peptide administration groups and the control groups, peripheral blood collected from three mice were pooled. To remove red blood cells, an equal volume of Hetasep (STEMCELL Technologies Inc., Cat No. ST-07906) as the collected blood was added, and centrifuged for two minutes at 100 G, incubated for 15 minutes at room temperature, and then the supernatant was collected. The supernatant was used in the next experiment as a sample containing nucleated cells found in peripheral blood.

iv) Colony assay

The supernatant (sample containing peripheral blood-derived cells) obtained by the above procedure was seeded on collagen I-coated 6-well plates (Corning, Cat No. 356400), and cultured for 10 days under the conditions of 37°C, 5% $CO_2$ and 5% $O_2$, using a medium containing Expansion Medium prepared using the MesenCult Expansion Kit (STEMCELL Technologies, Cat No. ST-05513) according to the manual of the kit, 1% L-glutamine (Nacalai Tesque Inc.), 10 $\mu$M ROCK inhibitor (Y27632, Tocris Bioscience) and 1% penicillin/streptomycin (Nacalai Tesque Inc.) (all numerical values are final concentrations). The medium was replaced with fresh medium twice a week during the culture period. On the 10th day of culture, cells on the plates were stained with a Differential Quik Stain Kit (Sysmex Corporation, Cat No. 16920), and the number of colonies containing 50 or more cells was counted.

In the experiments conducted by the present inventors so far, all the colonies obtained as a result of culturing peripheral blood on a solid phase such as a dish or a plate have adherability to the solid phase and have self-renewal ability. In addition, they have been confirmed to be PDGFR$\alpha$ positive, and to have the ability to differentiate into osteoblasts, chondrocytes, adipocytes, and epithelial cells.

In addition, the colonies obtained as a result of culturing, on a solid phase, peripheral blood after administration of a peptide consisting of amino acid residues 1-44 of the human HMGB1 protein (hereinafter, HMGB1 peptide 1-44), which has an activity of mobilizing mesenchymal stem cells into peripheral blood, have been confirmed to have adherability to solid phase and self-renewal ability, and to be PDGFR$\alpha$ positive. They have also been confirmed to have a gene expression profile characteristic to mesenchymal stem cells, based on the results of clustering the transcriptome analysis data and performing gene ontology analysis.

Furthermore, it has been also confirmed that the number of colonies obtained by solid-phase culture is larger in the peripheral blood after administration of HMGB 1 peptide 1-44 than in the peripheral blood after administration of physiological saline.

Therefore, the colonies obtained as a result of culturing peripheral blood on a solid phase are mesenchymal stem cells, and it is considered that an increase in the number of colonies detected in a solid phase culture of peripheral blood indicates an increase in the number of mesenchymal stem cells in the peripheral blood.

In addition, since usually mesenchymal stem cells are rarely present in peripheral blood, it is thought that the increased amount of mesenchymal stem cells was mobilized into peripheral blood from tissues other than peripheral blood (for example, bone marrow).

From the above, the number of colonies detected in the solid-phase culture of peripheral blood after administration of a test substance can be used as an indicator of the activity of the test substance to mobilize mesenchymal stem cells into peripheral blood.

After administering the test substance, the timing (peak time) at which the number of mesenchymal stem cells in the peripheral blood reaches the maximum is considered to vary among individuals. In the above-mentioned peripheral blood colony assay, there may be many cases where the timing of blood sampling (16 hours after test substance administration, in the case of 1r10 and its fragment peptides) does not match with the peak time of the number of mesenchymal stem cells in the peripheral blood. Therefore, instead of averaging the results of multiple assays, it was decided that the test substance resulting in formation of colonies at a number exceeding a predetermined threshold even once will be determined as having an activity to mobilize MSC into blood.

Furthermore, based on the results of multiple colony assays performed by the present inventors so far, the number of colonies formed from the negative control (the physiological saline or 1% DMSO administration group) has been confirmed to be "less than 10" no matter how many times the assay is performed. Therefore, in this Example, the number of colonies used as a threshold value was set to "10" colonies, and the test substance that resulted in formation of " 10 or more" colonies even once out of the 4 to 6 times the assay was performed was determined to be a substance having an activity to mobilize MSC into blood.

(2) Results

As a result of performing the assay 4 to 6 times for the peptides shown in Table 2, regarding the fragment peptides on the N-terminal side, 10 or more colonies were formed from the groups administered with 1r10_N03-N07, N11-12, N14, and MF5, and the number of colonies formed was less than 10 for the group administered with 1r10_N15 (Fig. 1). Regarding the fragment peptides on the C-terminal side, 10 or more colonies formed from the groups administered with 1r10_C05, C10-12, and C15-C17, and the number of colonies formed was less than 10 for the group administered with 1r10_C18 (Fig. 2). These results indicate that the amino acid sequences of 1r10_MF5 and 1r10_C17 are important core sequences for maintaining the activity to mobilize MSC into blood.

Reference Example 1

Efficacy of artificial sequence peptide 1r10 for inflammatory bowel disease

(1) Materials and methods

i) Drug

Dextran sodium sulfate (DSS) (molecular weight 36,000 to 50,000, manufactured by MP Biomedicals, Product No. 160110) was dissolved in purified water to prepare a 2.5% (w/v) DSS aqueous solution. In addition, the peptide 1r10 (TFA salt) consisting of the amino acid sequence of SEQ ID NO: 1 was chemically synthesized by the solid phase method and used as the test substance.

ii) Generation of inflammatory bowel disease (IBD) model mice

BALB/c mice (9 weeks old, male) were allowed to drink the 2.5% DSS aqueous solution freely for 7 days to induce colitis. After that, the drinking was changed from DSS to tap water, and the large intestine was collected 3 days later (10 days after the start of drinking the DSS aqueous solution).

iii) Peptide administration

The IBD model mice prepared as described above were divided into the peptide 1r10 administration group (n = 8) and the control group (n = 8). Peptide administration was carried out by injecting into the tail vein, the peptide 1r10 solution adjusted to a concentration of 0.5 mg/mL using physiological saline as a solvent at an amount of 10 mL/kg on Days 1, 4, 7, 8 and 9 after the start of drinking the DSS aqueous solution (peptide dose of 5 mg/kg). The control group was injected with physiological saline solution at a dose of 10 mL/kg into the tail vein on Days 1, 4, 7, 8 and 9 after the start of drinking the DSS aqueous solution.

iv) Evaluation of the effect of peptide administration

For 10 days from the start of drinking the DSS aqueous solution, the mice were weighed and observed for the symptoms (stool condition and bleeding) every day except Saturdays, Sundays, and holidays, and the degree of symptoms was evaluated by calculating the DAI (disease activity index) score. The DAI score was calculated by scoring the weight loss rate, stool condition, and stool bleeding as shown in the table below, and summing the scores of these three items. The scoring criteria were partly based on the method of Li et al. (PLoS One. 2015 Dec 7; 10 (12): e0144101).

[Table 3]
DAI (disease activity index) scoring

| Item | | Score |
|---|---|---|
| Rate of weight loss | No reduction | 0 |
| | 1-5% | 1 |
| | 5-10% | 2 |
| | 10-20% | 3 |

(continued)

DAI (disease activity index) scoring

| | Item | Score |
|---|---|---|
| | >20% | 4 |
| Stool condition | Normal | 0 |
| | Loose stool | 2 |
| | Diarrhea or watery stool | 4 |
| Bleeding | Absent | 0 |
| | Bloody stool | 1 |
| | Bloody stool (overall) | 2 |
| | Bleeding from the rectum | 4 |

In addition, the length of the large intestine (from the colon to the rectum) collected 10 days after the start of drinking DSS was measured using a digital caliper.

(2) Results

Figure 3 shows the changes in mouse body weight during the test period (see "saline" for the control group and "1r10" for the peptide-administered group). Both the control group and the peptide-administered group lost their body weight over time after the start of drinking DSS, but the degree of decrease was slower in the peptide-administered group than in the control group, and 10 days after the start of drinking the DSS aqueous solution, the peptide-administered group showed a tendency to recover body weight.

The stool score (sum of the "stool condition" score and the "bleeding" score scored as shown in Table 3 above) and the DAI score 8 to 10 days after the start of drinking the DSS aqueous solution are shown in Figures 4 and 5, respectively (see "saline" for the control group and "1r10" for the peptide-administered group). Both the stool score and the DAI score were lower in the peptide-administered group than in the control group.

Ten days after the start of drinking the DSS aqueous solution, the colon length of the peptide-administered group was longer than that of the control group (Figure 6; see "saline" for the control group and "1r10" for the peptide-administered group).

In IBD, inflammation occurs in the mucous membrane of the intestinal tract, erosions and ulcers are formed, and symptoms such as narrowing and shortening of the intestinal tract may occur. Another symptom of IBD is known to be occurrence of body weight loss, and the reason is thought to be malnutrition due to inflammation and tissue damage (such as ulcer) that occur in the intestinal mucosa. Furthermore, intravenous injection of mesenchymal stem cells in an animal IBD model is known to improve various symptoms including body weight loss, epithelial damage in the intestinal tract, infiltration of inflammatory cells, and such. Such improvement of symptoms is due to suppression of inflammation of the intestinal mucosa by the anti-inflammatory effect of mesenchymal stem cells, and resulting facilitation of mucosal tissue regeneration, and such.

By administering the artificial sequence peptide of the present application to the IBD model mice, body weight loss was suppressed, reduction of colon length was suppressed, and DAI score was improved. This is considered to be a result of the mesenchymal stem cells being mobilized into the peripheral blood by the action of the artificial sequence peptide of the present application, and the cells exerting the anti-inflammatory effect and the tissue regeneration effect.

Reference Example 2

Efficacy of the artificial sequence peptide 1r10 for atopic dermatitis

(1) Materials and methods

i) Drug

MC903 (generic name: Calcipotriol) was used to induce atopic dermatitis (hereinafter, also referred to as "AD") in mice. In addition, the peptide 1r10 (TFA salt) described in Reference Example 1 was used as the test substance.

ii) Generation of atopic dermatitis model mice

C57BL/6 mice (C57BL/6JJcl, 7-8 weeks old, male, microbial grade SPF) were obtained from Japan Claire Co., Ltd., acclimated in the animal breeding room for 5 days or more, and then subjected to the following experiments. An ethanol-adjusted MC903 solution was applied to the skin of the auricle of the mice at a dose of 2.25 nmol MC903/ear/once/day five times a week for 2 weeks (ten times in total) to induce atopic dermatitis (AD). This MC903-induced AD model is a model in which AD-like pathology can be reproduced in a short period of time (1-2 weeks) by artificially inducing the secretion of TSLP from epithelial cells, which is actually seen in AD patients, thereby eliciting a series of type 2 immune response cascades (Li M et al., Proc Natl Acad Sci USA. 2006; 103: 11736-41.). In addition, as a preliminary test, the appearance and tissue of the auricle were observed in normal mice and MC903-induced AD mice (14 days after the start of induction), and as a result the induced AD mice were found to develop erythema due to inflammation, scaling in the marginal area, and keratinous lichenization, epidermal acanthosis (thickening of the epidermis due to spinous cell proliferation), destruction of local barriers, infiltration of immune cells into the dermis, increased dermis thickness, and spongiosis in parts of the epidermis.

iii) Peptide administration

Two weeks (Day 15) after the start of application of MC903 (referred to as Day 1), only individuals in which AD was clearly induced judging from the main symptoms of scaling, erythema, and edema were selected. They were then divided into the control group and the peptide administration group (n = 7 in each group), while the groups were equalized based on the auricle thickness in consideration of the difference in the severity of inflammation between individuals. For peptide administration, the solution of peptide 1r10 adjusted to 0.3 mg/mL with physiological saline was injected into the tail vein at a volume of 5 mL/kg (peptide dose of 1.5 mg/kg) twice in total, once on the grouping day (Day 15) and once during the following 4 days (Day 16 to Day 19). For the control group a solution of bovine serum albumin (BSA) adjusted to 0.3 mg/mL with physiological saline was injected into the tail vein at a volume of 5 mL/kg (BSA dose of 1.5 mg/kg) twice in total, once on the grouping day (Day 15) and once during the following 4 days (Day 16 to Day 19).

iv) Evaluation of the effect of peptide administration

Evaluation of edema and scaling

During the peptide administration period (Day 15 to Day 19), ear swellings were measured daily to determine edema. Scaling, which is the main symptom of AD, was determined by photography (three times a week) using a digital camera (E-M1 II; Olympus) from a fixed base at a height of 13-14 cm from the auricle.

Pathological analysis

On the 4th day (Day 19) after the start of peptide administration, mice were placed under the influence of isoflurane anesthesia and euthanized with cervical spine dislocation. The skin at auricular inflammation site and its surroundings was quickly excised and collected while on ice to prevent protein denaturation of the sample. After removing excess hair and skin with tweezers, the collected auricular skin was immersed in 10% formalin and fixed at 4°C overnight while shaking on ice using a shaker. After fixation, the skin was thoroughly washed with phosphate buffered saline (PBS) and trimmed, and then a paraffin block was made using an automatic embedding device (Excelsior ES; Thermo Scientific) with attention to the tissue polarity in the dorsoventral axis/left-right axis specific to the skin). Then, it was sliced (5 μm thick) with a rotary microtome (HM 325; Thermo Scientific), fixed on a slide glass, and subjected to various staining. As pathological analysis, skin thickness and infiltration of immune cells were evaluated by histochemical staining and fluorescent immunostaining, respectively.

Measurement of dermis thickness

To quantitatively evaluate the healing condition of the site of inflammation, hematoxylin-eosin (HE) staining of the paraffin thin sections (5 μm thick) of skin tissue generated as described above was performed to measure the dermal thickness. Specifically, three sections were prepared for each individual, and each section was photographed with a microscope (Keyence BZ-X710) at one site each of the center of inflammation, head side, and tail side (that is, a total of 9 sites were photographed per individual). Next, for the photographed image, perpendicular lines were drawn from the epidermis-dermis junction to the subcutaneous fat-cartilage junction, and only the longest perpendicular line in the visual field was selected and used as the measured value of dermis thickness.

Quantification of immune cells

In order to quantitatively evaluate the number of immune cells at the site of inflammation, fluorescent immunostaining was performed on the paraffin slice (5 µm thick) prepared as described above using an anti-Laminin antibody and anti-CD45 antibody (for the detection of Laminin, Anti-Laminin (Sigma Aldrich; Catalog No. L9393) was used as the primary antibody, and Donkey anti-Rabbit IgG (H + L), Alexa Fluor 488 (Thermo Fisher Scientific; Catalog No. A-21206) was used as the secondary antibody. For the detection of CD45, Goat Anti-mouse CD45 (R&D Systems; Catalog No. AF114) was used as the primary antibody and Cy3 AffiniPure Bovine Anti-Goat IgG (H + L) (Jackson ImmunoReseach; Catalog No. 805-165-180) was used as the secondary antibody. For details of quantification, three sections were prepared for each individual, and each section was photographed using a microscope (Keyence BZ-X710) under high-power field (HPF: 400x) at one site each of the center of inflammation, head side, and tail side (that is, a total of 9 sites were photographed per individual). Next, for the photographed image, the CD45-positive cells (detected by Cy3 fluorescence) in the view field were counted and used as the measured value.

Statistical processing

The measured values obtained by the above analysis or the calculated values after correction were analyzed / judged after each statistical value was calculated using statistical analysis software (statcel-3 (OMS Publishing) and Excel statistics (BellCurve)). Specifically, as a preliminary test, (1) normality test (D'Agostino test with skewness/kurtosis coefficient; P> 0.05 is regarded as a normal distribution) and (2) rejection test (Grubbs test; P <0.05 is regarded as an outlier) were performed; and when the result obtained in (1) showed a normal distribution, t-test was performed between the groups. When the result did not show a normal distribution, Mann-Whitney U-test was performed as a nonparametric test based on the ranks between the groups, and a significant difference was determined to be present if p <0.05. For the changes in the body weight and auricle thickness, which were mainly measured repeatedly in time series, the repeated one-way ANOVA test was performed, and in addition the Tukey-Kramer test was performed as a post hoc test. Regarding the processing of outliers, the following method was employed: (a) if the number of individuals in the group with P <0.05 in the Grubbs test was 10% or less of the total number of individuals (n) in the group, the outliers were treated as missing values, and (b) if the number of individuals with P <0.05 exceeded 10% of the number n, they were imputed with the mean value of the other individuals with no outliers (so-called mean substitution).

(2) Results

Scaling

In the auricle of the control group mice after the induction of the AD model, scaling due to exfoliated epidermis caused by eczema and dryness, which are typical AD images, frequently occurred at the marginal part (Figure 7, arrow part in the left photograph). On the other hand, in the peptide-administered group, reduction of scaling in the auricle was observed (Figure 7, arrow part in the right photograph).

Auricular thickness (edema)

The transition of auricular thickness (edema) during the peptide administration period is shown in Figure 8 (see "Control" for the control group and "1r10" for the peptide administration group). A statistically significant reduction in auricular thickness was observed in the peptide-administered group as compared with the control group.

Dermis thickness

As a result of HE staining of skin tissue sections, in the control group, an increase in dermis thickness not seen in the AD non-induced mice (possibly due to cell infiltration and increased vascular permeability associated with the immune response caused by AD) was observed (Figure 9; see "Control" for the control group and "1r10" for the peptide-administered group). On the other hand, in the peptide-administered group, a statistically significant decrease in dermis thickness was observed as compared with the control group (Figure 10; see "Control" for the control group and "1r10" for the peptide-administered group). In addition, when peptide 1r10 was administered at a dose of 0.5 mg/kg, a statistically significant decrease in dermis thickness was observed as compared with the control group (data not shown).

Infiltration of immune cells

As a result of fluorescent immunostaining on skin tissue sections, in the control group, the tissue infiltration of CD45-positive immune cells associated with the immune response caused by AD was observed throughout the skin including around blood vessels (labeled with Laminin) (the arrow part in Figure 11; see "Control" for the control group and "1r10" for the peptide-administered group). On the other hand, in the peptide-administered group, a statistically significant decrease in the number of CD45-positive cells was observed as compared with the control group (Figure 12; see "Control" for the control group and "1r10" for the peptide-administered group).

The changes in mouse body weight during the period of this study (from the start of administration (Day 15) to the time of skin collection (Day 19)) were not significantly different between the groups and statistically significant changes were not observed between the groups ($p > 0.05$, repeated one-way ANOVA).

In this Reference Example, administration of the artificial sequence peptide of the present application to atopic dermatitis model mice suppressed scaling and edema, suppressed the increase in dermis thickness, and suppressed the infiltration of CD45-positive cells. This is considered to be a result of the mesenchymal stem cells being mobilized into the peripheral blood by the action of the artificial sequence peptide of the present application and the anti-inflammatory effect exhibited by the cells.

Reference Example 3

Efficacy of the artificial sequence peptide 1r10 for cerebral infarction

(1) Materials and methods

i) Drug

The peptide 1r10 (TFA salt) described in Reference Example 1 was used as the test substance.

ii) Generation of cerebral infarction model rat

Crl:CD (SD) rats (7 weeks old, male) were obtained from Charles River Laboratories, Japan, and after 6 days of acclimation, they were divided into the control group (n = 8) and the peptide-administered group (n = 8). On the following day of grouping, the rats were fixed in the supine position under 2% isoflurane inhalation anesthesia (anesthesia background: laughing gas:oxygen = 7:3). To regulate body temperature during surgery, a thermometer probe connected to a digital thermometer was inserted into the rectum, and changes in the rectal temperature before and after surgery were monitored. As a result, no decrease in rectal temperature was observed in any of the rats, and thus no heating was performed. The right common carotid artery, right external carotid artery and right internal carotid artery were exposed, and the right common carotid artery and right external carotid artery were ligated with sutures. A No. 4 nylon thread (embolus) pre-coated with silicon and cut to a length of 19 mm was inserted from the bifurcation of the right external carotid artery and the right internal carotid artery to occlude the right middle cerebral artery (MCA). After occlusion of the right MCA, the cervical skin was sutured and the rats were released from anesthesia. Thirty minutes after the right MCA occlusion, flexion of the forelimb contralateral to the occluded side was confirmed. Under inhalation anesthesia of 2% isoflurane, the right common carotid artery, right external carotid artery, and right internal carotid artery were exposed again, and 90 minutes after the right MCA occlusion, the embolus was removed to resume blood flow in the right MCA. After resuming blood flow in the right MCA, the right internal carotid artery was ligated and the cervical skin was sutured, and then the animals were released from anesthesia. Prior to the right MCA occlusion surgery, potassium benzylpenicillin was intramuscularly administered at a dose of 20000 units/kg.

iii) Peptide administration

Peptide administration was performed by injecting into the tail vein, 2 mL/kg of a peptide 1r10 solution adjusted to a concentration of 1 mg/mL using physiological saline solution as a solvent (peptide dose of 2 mg/kg) at 105 minutes and 24 hours after the right MCA occlusion. The control group was injected at the tail vein with 2 mL/kg physiological saline solution 105 minutes and 24 hours after the right MCA occlusion.

iv) Evaluation of the effect of peptide administration

Calculation of cerebral infarction volume ratio

Forty-eight hours after the right MCA occlusion, the animals were decapitated under anesthesia with sodium pentobarbital (50 mg/kg, intraperitoneal administration), and the entire brain was excised to prepare brain sections with a thickness of 2 mm. Brain sections were made at positions where coronal planes at 4 mm anterior to bregma, at 2 mm anterior to bregma, at bregma, at 2 mm posterior to bregma, at 4 mm posterior to bregma and at 6 mm posterior to bregma were obtained, with reference to Paxinos and Watson brain atlas (Paxinos G. and Watson C., The rat brain in stereotaxic coordinates, second edition. Academic Press Inc.; 1986). Brain sections were immersed and stained in 1 w/v% 2,3,5-Triphenyltetrazolium chloride (TTC) solution at room temperature and photographed. The resulting photographs were subjected to image analysis to obtain actual measurements of the cerebral infarction area and cross-sectional area of the brain, and the cerebral infarct volume and the total brain volume from 4 mm anterior to bregma to 6 mm posterior to bregma were calculated using the calculation formula below. In addition, the cerebral infarction volume ratio was calculated from the cerebral infarction volume and the total volume of the brain (cerebral infarction volume ratio (%) = cerebral infarction volume / total brain volume x 100).

$$V = 2 (a + b + \sqrt{ab})/3 + 2 (b + c + \sqrt{bc})/3 + 2 (c + d + \sqrt{cd})/3 + 2 (d + e + \sqrt{de})/3 + 2 (e + f + \sqrt{ef})/3$$
$$= 2 (a + f + \sqrt{ab} + \sqrt{bc} + \sqrt{cd} + \sqrt{de} + \sqrt{ef})/3 + 4 (b + c + d + e)/3$$

V: Cerebral infarction volume or total brain volume ($mm^3$)
a: Cerebral infarct area or cross-section area of the brain on the cross section of 4 mm anterior to bregma ($mm^2$)
b: Cerebral infarct area or cross-section area of the brain on the cross section of 2 mm anterior to bregma ($mm^2$)
c: Cerebral infarct area or cross-section area of the brain on the cross section of bregma ($mm^2$)
d: Cerebral infarct area or cross-section area of the brain on the cross section of 2 mm posterior to bregma ($mm^2$)
e: Cerebral infarct area or cross-section area of the brain on the cross section of 4 mm posterior to bregma ($mm^2$)
f: Cerebral infarct area or cross-section area of the brain on the cross section of 6 mm posterior to bregma ($mm^2$)

(2) Results

The cerebral infarction volume ratios of the control group and the peptide-administered group were 34.1 $\pm$ 1.1% and 27.2 $\pm$ 1.2% (mean $\pm$ standard error), respectively (Figure 13; see "Control" for the control group and "1r10" for the peptide-administered group). The cerebral infarction volume ratio in the peptide-administered group was lower than that in the control group, and a statistically significant difference was observed (Mann-Whitney U test, $p < 0.01$).

In this Reference Example, the effect of reducing the cerebral infarction lesion was obtained by administering the artificial sequence peptide of the present application to a cerebral infarction model rat. This is considered to be a result of the mesenchymal stem cells being mobilized into the peripheral blood by the action of the artificial sequence peptide of the present application, and the cells exerting an anti-inflammatory effect, a trophic effect (secretion of trophic factors), a tissue regeneration effect, and such.

[Industrial applicability]

The peptide of the present application can be used as a composition for mobilizing mesenchymal stem cells into peripheral blood, and as a therapeutic agent for inflammatory diseases, autoimmune diseases, fibrotic diseases, and diseases accompanied by tissue damage/ischemia/necrosis.

**Claims**

1. A composition for use in mobilizing mesenchymal stem cells into peripheral blood, which comprises a peptide selected from the following:

   (a) a peptide consisting of a portion of the amino acid sequence of SEQ ID NO: 1, and comprising the amino acid sequence of SEQ ID NO: 17 or 19;
   (b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and
   (c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

2. A composition for use in treatment of a disease or pathological condition in a subject by mobilizing mesenchymal stem cells into peripheral blood, which comprises a peptide selected from the following:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

3. The composition of claim 2, wherein the treatment of a disease or pathological condition is selected from inflammation-suppressing therapy, immunomodulatory therapy, tissue regeneration-inducing therapy, and tissue fibrosis-suppressing therapy.

4. The composition of claim 2, wherein the disease or pathological condition is selected from an inflammatory disease, an autoimmune disease, a disease accompanied by tissue damage, ischemia, or necrosis, and a fibrotic disease.

5. The composition of claim 2, wherein the disease or pathological condition is inflammatory bowel disease.

6. The composition of claim 2, wherein the disease or pathological condition is ulcerative colitis.

7. The composition of claim 2, wherein the disease or pathological condition is atopic dermatitis.

8. The composition of claim 2, wherein the disease or pathological condition is cerebral infarction.

9. A composition for use in treatment of a disease selected from inflammatory bowel disease, atopic dermatitis, and cerebral infarction, which comprises a peptide selected from the following:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

10. A peptide selected from the following:

(a) a peptide consisting of a portion of the amino acid sequence of SEQ ID NO: 1, and comprising the amino acid sequence of SEQ ID NO: 17 or 19;

(b) a peptide consisting of all or a portion of an amino acid sequence resulting from substitution, deletion, insertion, or addition of one to five amino acids in the amino acid sequence of SEQ ID NO: 1, wherein the peptide comprises the amino acid sequence of SEQ ID NO: 17 or 19; and

(c) a peptide consisting of all or a portion of an amino acid sequence having a sequence identity of 90% or higher with the amino acid sequence of SEQ ID NO: 1, wherein the peptide comprises the amino acid sequence of SEQ ID NO: 17 or 19.

FIG. 1

FIG. 2

FIG. 3

FIG. 4

FIG. 5

FIG. 6

Control                    1r10

# FIG. 7

Ear Swellings change (%) [means + S.E. (n=7)]   (Left Ear)

FIG. 8

Control

1r10

FIG. 9

FIG. 10

Control

1r10

FIG. 11

FIG. 12

FIG. 13

## INTERNATIONAL SEARCH REPORT

| International application No. |
| --- |
| PCT/JP2021/014253 |

A. CLASSIFICATION OF SUBJECT MATTER
A61K 38/16(2006.01)i; A61P 1/04(2006.01)i; A61P 9/10(2006.01)i; A61P 17/00(2006.01)i; A61P 29/00(2006.01)i; A61P 37/02(2006.01)i; A61P 37/08(2006.01)i; A61P 43/00(2006.01)i; C07K 7/08(2006.01)i; C07K 14/00(2006.01)i
FI:      A61K38/16; A61P1/04; A61P9/10; A61P17/00; A61P29/00; A61P37/02; A61P37/08; A61P43/00 105; C07K7/08; C07K14/00 ZNA

According to International Patent Classification (IPC) or to both national classification and IPC

B. FIELDS SEARCHED

Minimum documentation searched (classification system followed by classification symbols)
A61K38/16; A61P1/04; A61P9/10; A61P17/00; A61P29/00; A61P37/02; A61P37/08; A61P43/00; C07K7/08; C07K14/00

Documentation searched other than minimum documentation to the extent that such documents are included in the fields searched
Published examined utility model applications of Japan       1922–1996
Published unexamined utility model applications of Japan     1971–2021
Registered utility model specifications of Japan             1996–2021
Published registered utility model applications of Japan     1994–2021

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN); UniProt/GeneSeq

C. DOCUMENTS CONSIDERED TO BE RELEVANT

| Category* | Citation of document, with indication, where appropriate, of the relevant passages | Relevant to claim No. |
| --- | --- | --- |
| A | WO 2011/052668 A1 (GENOMIX CO., LTD.) 05 May 2011 (2011-05-05) claims, paragraph [0005], examples | 1–10 |
| A | WO 2009/133939 A1 (GENOMIX CO., LTD.) 05 November 2009 (2009-11-05) claims, paragraph [0010], examples | 1–10 |

☒ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 May 2021 (06.05.2021) | 18 May 2021 (18.05.2021) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

| International application No. |
| --- |
| PCT/JP2021/014253 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | 何彦霆ほか，"HMGB1 ameliorates inflammatory bowel disease by inducing circulating mesenchymal stem cells"，第17回日本再生医療学会総会 プログラム抄録，22 March 2018, 0-34-2, [retrieval date 21 October 2019], Internet: \<URL:http://www2.convention.co.jp/17jsrm/appli/\>, entire text, (Ho, Yenting, et al., Programs and abstracts of the 17th congress of the Japanese society for regenerative medicine) | 1-10 |
| A | YAMAOKA, S. et al., "Systemic delivery of HMGB1 peptide ameliorates imiquimod-induced psoriasis-like dermatitis", Journal of Investigative Dermatology, May 2018, vol. 138, issue 5, supplement, p. S177, 1043, entire text | 1-10 |
| A | WO 2018/186480 A1 (STEMRIM INC.) 11 October 2018 (2018-10-11) claims, paragraph [0006], examples | 1-10 |
| A | WO 2019/107530 A1 (STEMRIM INC.) 06 June 2019 (2019-06-06) claims, paragraph [0006], examples | 1-10 |
| P, X | WO 2020/071520 A1 (STEMRIM INC.) 09 April 2020 (2020-04-09) claims, examples | 1-10 |
| P, X | WO 2020/158924 A1 (OSAKA UNIVERSITY) 06 August 2020 (2020-08-06) example 6 | 1-10 |

Form PCT/ISA/210 (continuation of second sheet) (January 2015)

**INTERNATIONAL SEARCH REPORT**
Information on patent family members

International application No.

PCT/JP2021/014253

| Patent Documents referred in the Report | Publication Date | Patent Family | Publication Date |
|---|---|---|---|
| WO 2011/052668 A1 | 05 May 2011 | US 2012/0251510 A1 claims, examples EP 2494977 A1 CN 102711777 A KR 10-2012-0135190 A | |
| WO 2009/133939 A1 | 05 Nov. 2009 | US 2011/0091928 A1 claims, examples EP 2301559 A1 KR 10-2011-0028440 A CN 102076350 A | |
| WO 2018/186480 A1 | 11 Oct. 2018 | US 2020/0038486 A1 claims, examples EP 3607963 A1 CN 110621331 A KR 10-2019-0135519 A | |
| WO 2019/107530 A1 | 06 Jun. 2019 | US 2020/0384074 A1 claims, examples CN 111542335 A | |
| WO 2020/071520 A1 | 09 Apr. 2020 | (Family: none) | |
| WO 2020/158924 A1 | 06 Aug. 2020 | (Family: none) | |

Form PCT/ISA/210 (patent family annex) (January 2015)

## REFERENCES CITED IN THE DESCRIPTION

**Patent documents cited in the description**

- WO 2008053892 A
- WO 2009133939 A
- WO 2012147470 A
- JP 2019039232 W

**Non-patent literature cited in the description**

- *PNAS,* 19 April 2011, vol. 108 (16), 6609-14
- *Gene,* 1988, vol. 67, 31-40
- *Bio/Technology,* 1988, vol. 6, 47-55
- **MARSHAK et al.** Strategies for Protein Purification and Characterization: A Laboratory Course Manual. Cold Spring Harbor Laboratory Press, 1996
- Remington's Pharmaceutical Science. Mark Publishing Company
- **LI et al.** *PLoS One.,* 07 December 2015, vol. 10 (12), e0144101
- **LI M et al.** *Proc Natl Acad Sci USA.,* 2006, vol. 103, 11736-41
- **PAXINOS G ; WATSON C.** The rat brain in stereotaxic coordinates. Academic Press Inc, 1986